# EUROPÄISCHE PATENTANMELDUNG

(11) **EP 1 628 124 A2**
(43) Veröffentlichungstag der Anmeldung: **22.02.2006**
(21) Anmeldenummer: 05107232.0
(22) Anmeldetag: 05.08.2005
(51) Int. Cl.: G01N 15/08, G01N 33/34, G01N 33/36

(54) **Vorrichtung zur Bestimmung der Porosität einer Faserstoffbahn**

(30) Priorität: 20.08.2004 DE 102004040426
(71) Anmelder: Voith Paper Patent GmbH, 89522 Heidenheim (DE)
(72) Erfinder: Berger, Susanne, 89518, HEIDENHEIM (DE)

(57) **Zusammenfassung**

Eine Messanordnung (1) zur Bestimmung eines Absolutwertes der Porosität und zur Bestimmung der Porositätsverteilung einer Papier-, Karton-, Tissue- oder einer anderen Faserstoffbahn, ist dadurch gekennzeichnet, dass sie eine Haltevorrichtung zum Festhalten eines Bereichs (2) der Faserstoffbahn, eine Durchflusseinrichtung mit einer Zuführvorrichtung (4) zum Zuführen eines ersten Hilfsstoffes auf die Oberseite des Bereichs (2), wobei der erste Hilfsstoff flächenförmig innerhalb des Bereichs auf die Faserstoffbahn einwirkt, und eine Messeinrichtung (9) zum Messen von durch die Faserstoffbahn hindurchströmenden Substanzen aufweist.

## Beschreibung

Die Erfindung bezieht sich auf eine Messanordnung zur Bestimmung eines Absolutwertes der Porosität einer Papier-, Karton-, Tissue- oder einer anderen Faserstoffbahn.

In Maschinen zur Herstellung einer Papier-, Karton-, Tissue- oder einer anderen Faserstoffbahn wird einer in einer Siebpartie aus einer Faserstoffsuspension formierten Faserstoffbahn in einer Pressanordnung und dann in einer Trockenanordnung weitere Feuchtigkeit entzogen. Allerdings tritt bei der Produktion von Faserstoffbahnen immer wieder das Problem auf, dass sogenannte "Feuchtigkeitsnester" in und zwischen den Fasern der Bahn zurückbleiben, die durch den normalen Trocknungsvorgang über die Trockenzylinder nicht beseitigt werden, da die Trockenzylinder nur eine für die gesamte Fläche der Faserstoffbahn gleichmäßige Trocknungsleistung zur Verfügung stellen. Die Feuchtigkeitsnester haben einen Durchmesser im Zentimeterbereich, der bis zum Durchmesser einer Münze (20 mm) heranreicht. Die Feuchtigkeitsnester wirken sich besonders bei der Satinage der Bahn negativ aus, bei der nach hoher Temperatur- und Druckeinwirkung das sogenannte "Cockling" entsteht.

Das Cockling ist ein insbesondere bei relativ dichten holzhaltigen Papieren auftretendes Flachlageproblem. Es entsteht vor allem beim Kalandrieren und wird auf lokal unterschiedliche Feuchtegehalte zurückgeführt. Diese entstehen vermutlich bei dichten Papieren durch lokal unterschiedliche Porositäten im Papier, die bei der Trocknung für das Entweichen der Feuchtigkeit verantwortlich sind.

Verschiedene weitere Qualitätsmerkmale, die auch bei der Papierweiterverarbeitung auftreten, beispielsweise Streichfarbenannahme und Mottling, lassen sich ebenfalls auf eine unterschiedliche Verteilung von Füllstoffen und Fasern oder auf eine lokal unterschiedliche Porosität im Papier zurückführen. Die Messung lokaler Porositäten mit hoher Auflösung erscheint somit als ein wichtiges Kriterium zur Erkennung von Qualitätsmängeln verschiedener Papiersorten.

Bisher wird die Porosität von Papier mittels eines Messgerätes nach Bendtsen bestimmt, bei dem ein Ring einer bestimmten Größe, beispielsweise mit einem Durchmesser von 50 mm, auf das Papier gepresst und der Luftdurchfluss gemessen wird. Dieser wird als Messgröße, beispielsweise in Millilitern pro Minute, ausgegeben. Jedoch ermöglicht diese Messmethode lediglich die Bestimmung der GesamtPorosität über einer Fläche von über 2000 m² und kann daher nicht zur Aufklärung über die Ursache von Qualitätsmängeln beitragen.

Andererseits wurde in neuerer Zeit ein Messverfahren entwickelt, das mittels einer Lochplatte und einer Messsonde die Bestimmung der lokalen Porositäten mit einer minimalen Auflösung von 1 mm² ermöglichen soll. Dieses Messverfahren hat jedoch den Nachteil, dass die Genauigkeit der Messwerte aufgrund hoher Reibungsverluste der hierbei eingesetzten Luft in einem Zuführschlauch und in einer Düse beschränkt ist und dass je nach Größe und Anpressdruck einer bei diesem Verfahren eingesetzten Lochplatte Luftverluste über der Papieroberfläche auftreten, die zu einer Verfälschung der Durchströmungsmesswerte führen. Es ist ein hoher Messaufwand erforderlich, da jeder Messpunkt einzeln bestimmt werden muss oder hohe Kosten für eine Vielzahl von Messsonden oder Umschaltventile entstehen.

Es ist die Aufgabe der Erfindung, eine Vorrichtung zur effizienten und hochauflösenden Bestimmung lokal unterschiedlicher Feuchtegehalte in einer Papierfläche zu schaffen.

Erfindungsgemäß wird die Aufgabe dadurch gelöst, dass sie eine Haltevorrichtung zum Festhalten eines Bereichs der Faserstoffbahn, eine Durchflusseinrichtung mit einer Zuführvorrichtung zum Zuführen eines ersten Hilfsstoffes auf die Oberseite des Bereichs, wobei der erste Hilfsstoff flächenförmig innerhalb des Bereichs auf die Faserstoffbahn einwirkt, und eine Messeinrichtung zum Messen von durch die Faserstoffbahn hindurchströmenden Substanzen aufweist.

Durch den Einsatz der Erfindung wird eine Messmethodik mit hoher Auflösung und geringem Investitionsaufwand geschaffen, mit der sich eine relative Verteilung der Porosität innerhalb einer größeren Fläche des Papiers bestimmen lässt und die zusätzlich einen absoluten Mittelwert über diese Fläche liefert. Die Erfindung ist ebenfalls geeignet zur Bestimmung der lokalen Messwerte der Porosität, d. h. der Porositätsverteilung.

Die Vorteile der Erfindung bestehen im wesentlichen darin, dass sich das bewährte Konzept der Messung der Durchströmung von Luft oder eines anderen Gases oder Gasgemisches aufrechterhalten lässt. Dadurch wird gewährleistet, dass die mit dem erfindungsgemäßen System gemessenen Werte sich mit denen vergleichen lassen, die nach dem bekannten Messverfahren nach Bendtsen gemessen werden. Bei einer einzelnen Messung lassen sich ein Absolutwert und die Verteilung der Porosität über der ganzen Fläche ermitteln. Es werden eine schnelle Messung und eine schnelle Auswertung ermöglicht, während gleichzeitig ein geringer Aufwand an Messtechnik verursacht wird. Gemäß der Erfindung kann für den einzusetzenden Hilfsstoff als Träger ebenfalls ein Gas oder ein flüssiger Träger, insbesondere Wasser, eingesetzt werden.

Weitere Vorteile der Erfindung bestehen darin, dass das bekannte Messverfahren nach Bendtsen weiterentwickelt wurde. Mit einer einzigen Messung werden zwei Informationen erzeugt: es wird die Gesamtporosität bestimmt, wie es auch nach dem Stand der Technik bereits möglich ist, und zusätzlich wird die Porositätsverteilung über der Fläche ermittelt. Es werden somit ein Absolutwert der Porosität über eine bestimmte Fläche und die Bestimmung der lokalen Messwerte der Porosität (Porositätsverteilung) realisiert.

Falls die bekannte Messapparatur nach Bendtsen als Luftströmungssystem verwendet wird, ist nur ein geringer Aufwand an zusätzlicher Technik erforderlich, um die Erfindung zu realisieren. Die bei den Messungen erzeugten Proben lassen sich zur weiteren Analyse verwenden. Die erfindungsgemäße Messapparatur lässt sich ohne zusätzlichen Entwicklungsaufwand auch ohne den Einsatz eines Computers, eines Scanners oder von Software zur visuellen Bewertung der Porositätsverteilung verwerten.

Vorteilhafte Weiterbildungen der Erfindung ergeben sich aus den Unteransprüchen, der Beschreibung und den Zeichnungen.

In einer vorteilhaften Ausgestaltung der Erfindung ist vorgesehen, dass die Haltevorrichtung eine fluiddichte Einspannvorrichtung umfasst.

In vorteilhafter Weise enthält die Messanordnung eine Durchflusseinrichtung, die ein Fluidströmungssystem mit wenigstens einer Einrichtung zum Zuführen eines Fluids, ein System zum Einbringen des ersten Hilfsstoffes und ein System zur Mischung oder Erzeugung einer homogenen Mischung des Fluids mit dem ersten Hilfsstoff umfasst.

Von Vorteil ist eine Ausgestaltung, in der die Messanordnung eine Einrichtung zum Einbringen mindestens eines zweiten Hilfsstoffes, insbesondere zur Abbildung eines Porositätsverteilungsbildes des Bereichs, insbesondere auf die Unterseite des Bereichs, aufweist. Vorteilhaft ist ebenfalls eine Ausgestaltung, in der eine Auswerteeinrichtung mit einem mit einer bildanalytischen Software ausgestatteten Rechner und einem Scanner umfasst.

Die Erfindung bezieht sich auch auf ein Verfahren zum Messen der Porosität mit der oben beschriebenen Messanordnung zur Bestimmung eines Absolutwertes der Porosität einer Papier-, Karton-, Tissue- oder einer anderen Faserstoffbahn.

Das Verfahren ist dadurch gekennzeichnet, dass der erste Hilfsstoff mittels eines Fluids oder eines Fluidgemischs oder direkt von der Oberseite auf einen Bereich der Faserstoffbahn aufgebracht wird, mit dem Stoff der Faserstoffbahn eine Reaktion eingeht und dass der Durchfluss des Fluids oder des Fluidgemischs zusammen mit dem in ihm verbliebenen ersten Hilfsstoff erfasst wird. Gemäß der Erfindung wird somit ein indirektes Messverfahren für die Messung der Porositätsverteilung in einer Materialbahn durch die Nutzung der Wirkung eines durch die Poren strömenden Fluids auf eine Reagenzschicht eingesetzt.

In vorteilhafter Weise wird das Verfahren so durchgeführt, dass der erste Hilfsstoff durch Färbung oder Entfärbung der Faserstoffbahn mit dem Stoff thermisch oder durch eine chemische Reaktion reagiert und dass die Färbung bzw. die Entfärbung der Faserstoffbahn gemessen wird.

In alternativer Weise kommt ein zweiter Hilfsstoff zusätzlich zum Einsatz, wobei wieder der erste Hilfsstoff mittels eines Fluids oder eines Fluidgemischs oder direkt von der Oberseite auf einen Bereich der Faserstoffbahn aufgebracht wird, der Durchfluss des Fluids oder des Fluidgemischs zusammen mit dem in ihm verbliebenen ersten Hilfsstoff erfasst wird und dann auf der Unterseite der Faserstoffbahn der zweite Hilfsstoff, insbesondere in flächenförmiger Form, zugesetzt wird bzw. vorliegt. Hierbei wird dann die Färbung oder Entfärbung des zweiten Hilfsstoffs durch den ersten Hilfsstoff gemessen.

Der Einsatz des ersten Hilfsstoffs allein oder in Verbindung mit dem zweiten Hilfsstoff erzeugt eine thermische Reaktion oder eine chemische Reaktion, die zu einer Färbung oder Entfärbung der Faserstoffbahn in dem untersuchten Bereich oder zu einer Färbung oder Entfärbung des zweiten Hilfsstoffs führt. Dieser wird beispielsweise entweder auf die Faserstoffbahn aufgetragen, etwa auf deren Unterseite, oder er bildet eine gesonderte, unter den Bereich der Faserstoffbahn gelegte Schicht oder ist in dieser enthalten. In jedem Fall wird die Schicht, in der die Farbreaktion stattgefunden hat, optisch ausgewertet.

Nachstehend wird die Erfindung in einem Ausführungsbeispiel näher erläutert. Es zeigen:
- Fig. 1: eine schematische Darstellung einer Messanordnung zur lokalen Porositätsmessung,
- Fig. 2a, b: einen schematischen Querschnitt durch einen Bereich der Faserstoffbahn, vor und nach dem Durchströmen eines mit dem ersten Hilfsstoff beaufschlagten Fluids oder Fluidgemischs und
- Fig. 3a, b: einen schematischen Querschnitt durch einen Bereich der Faserstoffbahn, vor und nach dem Durchströmen eines mit dem ersten Hilfsstoff beaufschlagten Fluids oder Fluidgemischs, wobei unterhalb des Bereichs eine mit dem zweiten Hilfsstoff ausgestattete Schicht angeordnet ist.

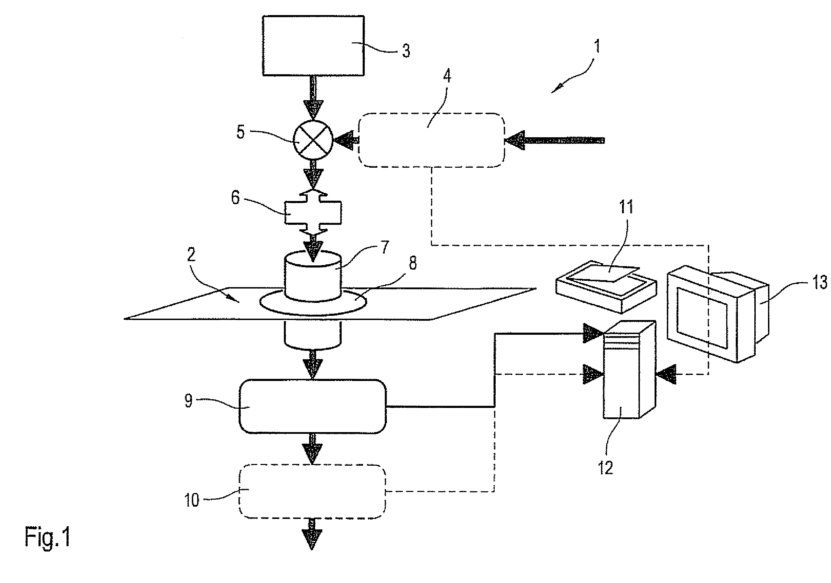

Eine Messanordnung1 (Fig. 1) zur Bestimmung der Porosität in einem repräsentativen Bereich 2 einer Faserstoffbahn umfasst einen Vorratsbehälter oder Erzeuger 3 (bspw. Ozon Generator) mit einem fluiden Medium, insbesondere mit Luft oder einem anderen Fluid, das unter Druck steht, sowie eine Zuführeinheit 4 für einen ersten Hilfsstoff, der beispielsweise in einem Fluid wie Wasser gelöst ist, oder zunächst in reiner Form in der Zuführeinheit 4 vorhanden ist und dann in einer Mischeinrichtung 5 mit dem Medium gemischt wird und über einen Strömungsgleichrichter 6 zur Vergleichmäßigung des den ersten Hilfsstoff umfassenden Fluidgemischs 1 durch das Zuströmgefäß 7 auf den Bereich 2 geleitet wird.

Auf der Unterseite des Bereiches 2 ist ein Durchflussmesser 9 angeordnet, der die Menge des Fluidgemisches bestimmt, das den Bereich in einer vorgegebenen Zeiteinheit durchströmt. Zusätzlich kann auch eine Messeinheit 10 zur Bestimmung der Konzentration des ersten Hilfsstoffs in dem durchströmenden Fluid vorhanden sein.

Während des Messvorgangs oder nach Beendigung des Messvorgangs wird die Färbung der Faserstoffbahn in dem Bereich 2 durch eine optische Messeinheit, insbesondere mittels eines Scanners 11, erfasst und anschließend und/oder parallel durch einen mit einer entsprechenden Software ausgestatteten Computer 12 ermittelt und an einem Monitor 13 dargestellt.

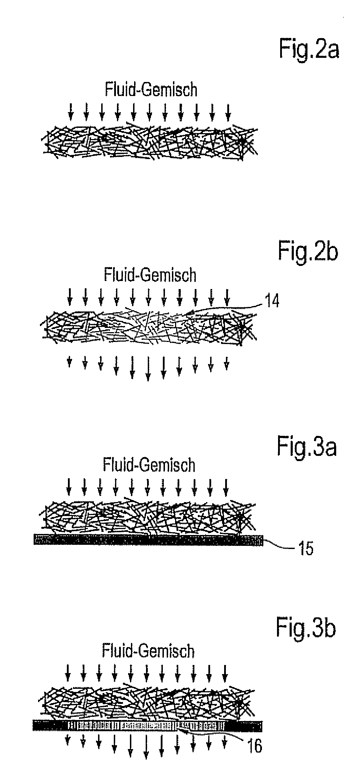

Bei der Strömung durch das Papier tritt eine Reaktion zwischen dem ersten Hilfsstoff und dem Papier auf, die je nach lokaler Durchflussmenge und Konzentration zu einer Färbung 14 (Fig. 2b) oder einer Entfärbung des Papiers führt, die durch eine oxidierende oder reduzierende Reaktion des ersten Farbstoffs mit dem Papier hervorgerufen wird. Anschließend wird der ganz oder teilweise entsprechend der Porositätsverteilung gefärbte oder entfärbte Bereich 2 mittels des Scanners 11 erfasst und die lokale Porositätsverteilung über der Fläche in einer Graustufenverteilung und des Maximalwerts der Porosität bildanalytisch ausgewertet.

Dieses Meßprinzip wird auf eine Versuchsdurchführung übertragen, in der der erste Hilfsstoff nicht unmittelbar mit dem Stoff der Faserstoffbahn reagiert, In diesem Fall wird auf der Unterseite der Faserstoffbahn in dem zu untersuchenden Bereich 2 eine Beschichtung mit einem zweiten Hilfsstoff aufgebracht, oder dieser wird in einer eigenen Schicht 15 (Fig. 3a) unter die Faserstoffbahn gelegt, so dass er sich während des Durchströmens des den ersten Hilfsstoff enthaltenden Fluids in einer Zone 16 (Fig. 3b) entsprechend der Porositätsverteilung färbt oder entfärbt.

Als Fluid für die genannte Versuchsanordnung ist auch Wasser denkbar. Der Effekt beruht bei dieser Anordnung z.B. auf der Auswaschung oder Oxidation des Farbstoffes des unterhalb des Papiers angeordneten Trägermaterials (Hilfstoff 15) welches sich je nach durchströmter Menge mehr oder weniger entfärbt und damit Auskunft über die Porositäten und die Entwässerungsmechanismen von Papierproben aus verschieden Positionen der Papiermaschine geben kann, da der simulierte Strömungsvorgang je nach Anforderung (Pressenpartie, Siebpartie) mit der Versuchsanordnung abgebildet werden kann. Die Papierproben können - sofern sie als Trägermaterial für die Reaktion dienen - weiteren Analysen, wie dem Splitting und/oder lokalen Aschebestimmungen zugeführt werden.

Durch ein Splitten (das Trennen der Papierprobe in verschieden Schichten) mittels Klebefolie (z.B. am VTT in Finnland oder an der Universität in Graz angewendete Methode zur Messung der Faseroreintieurng in Z-Richtung) können je nach Messanordnung bei einer Entfärbung des vorher angefärbten Papier bzw. einer Färbung des gemessenen Papiers die Strömungskanäle innerhalb des Papiers in z-Richtung visualisiert werden.

## Patentansprüche

1. Messanordnung (1) zur Bestimmung eines Absolutwertes der Porosität einer Papier-, Karton-, Tissue- oder einer anderen Faserstoffbahn,
**dadurch gekennzeichnet,**
**dass** sie eine Haltevorrichtung zum Festhalten eines Bereichs (2) der Faserstoffbahn, eine Durchflusseinrichtung mit einer Zuführvorrichtung (4) zum Zuführen eines ersten Hilfsstoffes auf die Oberseite des Bereichs (2), wobei der erste Hilfsstoff flächenförmig innerhalb des Bereichs auf die Faserstoffbahn einwirkt, und eine Messeinrichtung (9) zum Messen von durch die Faserstoffbahn hindurchströmenden Substanzen aufweist.

2. Messanordnung (1) nach Anspruch 1,
**dadurch gekennzeichnet,**
**dass** durch die Messanordnung in einer Messung ein Absolutwert der Porosität einer Fläche und lokale Messwerte der Porosität (Porositätsverteilung) dieser Fläche ermittelbar sind.

3. Messanordnung (1) nach Anspruch 1 oder 2,
**dadurch gekennzeichnet,**
**dass** die Haltevorrichtung eine fluiddichte Einspannvorrichtung (7, 8) umfasst.

4. Messanordnung (1) nach einem der Ansprüche 1 bis 3,
**dadurch gekennzeichnet,**
**dass** die Durchflusseinrichtung ein Fluidströmungssystem mit wenigstens einer Einrichtung (3) zum Zuführen eines Fluids, ein System zum Einbringen des ersten Hilfsstoffes und ein System (5, 8) zur Mischung oder Erzeugung einer homogenen Mischung des Fluids mit dem ersten Hilfsstoff umfasst.

5. Messanordnung (1) nach Anspruch 4,
**dadurch gekennzeichnet,**
**dass** sie eine Einrichtung zum Einbringen mindestens eines zweiten Hilfsstoffes, insbesondere zur Abbildung eines Porositätsverteilungsbildes des Bereichs, insbesondere auf die Unterseite des Bereichs, aufweist.

6. Messanordnung (1) nach einem der Ansprüche 1 bis 5,
**dadurch gekennzeichnet,**
**dass** sie eine Auswerteeinrichtung mit einem mit einer bildanalytischen Software ausgestatteten Rechner (12) und einem Scanner (11) umfasst.

7. Verfahren zum Messen der Porosität mit einer Messanordnung zur Bestimmung eines Absolutwertes der Porosität einer Papier-, Karton-, Tissue- oder einer anderen Faserstoffbahn nach einem der Ansprüche 1 bis 5,
**dadurch gekennzeichnet,**
**dass** der erste Hilfsstoff mittels eines Fluids oder eines Fluidgemischs oder direkt von der Oberseite auf einen Bereich (2) der Faserstoffbahn aufgebracht wird, mit dem Stoff der Faserstoffbahn eine Reaktion eingeht bzw. Ablagerungen bildet und dass der Durchfluss des Fluids oder des Fluidgemischs zusammen mit dem in ihm verbliebenen ersten Hilfsstoff erfasst wird.

8. Verfahren nach Anspruch 7,
**dadurch gekennzeichnet,**
**dass** der erste Hilfsstoff durch Färbung oder Entfärbung der Faserstoffbahn mit dem Stoff thermisch oder durch eine chemische Reaktion reagiert und dass die Temperaturverteilung durch eine Thermo-Kamera und / oder die Färbung und /oder die Entfärbung der Faserstoffbahn gemessen wird.

9. Verfahren nach Anspruch 8,
**dadurch gekennzeichnet,**
**dass** der erste Hilfsstoff mittels eines Fluids oder eines Fluidgemischs oder direkt von der Oberseite auf einen Bereich der Faserstoffbahn aufgebracht wird, dass der Durchfluss des Fluids oder des Fluidgemischs zusammen mit dem in ihm verbliebenen ersten Hilfsstoff erfasst wird, dass auf der Unterseite der Faserstoffbahn ein zweiter Hilfsstoff, insbesondere in flächenförmiger Form, zugesetzt wird und dass die Färbung oder Entfärbung des zweiten Hilfsstoffs durch den ersten Hilfsstoff gemessen wird.
